# EUROPEAN PATENT APPLICATION

(11) **EP 4 582 115 A2**
(43) Date of publication of application: **09.07.2025**
(21) Application number: 24213602.6
(22) Date of filing: 18.11.2024
(51) Int. Cl.: A61M 1/36

(54) **HEART-LUNG BYPASS MACHINE SYSTEM**

(30) Priority: 15.12.2023 US 202318541535
(71) Applicant: LivaNova Deutschland GmbH, 80939 Munich (DE)
(72) Inventor: SCHUBERT, Friedemann, 80639 München (DE); HERZOG, Helen, 85748 Garching (DE); KOHNEN, Lena, 81369 München (DE)
(74) Representative: Grünecker Patent- und Rechtsanwälte PartG mbB

(57) **Abstract**

An HLM system may include a controller and a plurality of venous sensors disposed along a venous pathway. Upon activation of the HLM system, the controller may be configured to suppress alarms associated with sensors until blood is detected within the venous pathway by a venous blood gas sensor. The controller may be configured to start a bypass timer upon detection of blood within the venous pathway. A method of bypassing a patient's heart with an HLM system may include activating the HLM system, coupling a venous pathway of the HLM system to at least one venous portion of a patient's vasculature; coupling an arterial pathway of the HLM system to at least one arterial portion of the patient's vasculature; and suppressing alarms associated with sensors until blood is detected within the venous pathway by the venous blood gas sensor.

## Description

### TECHNICAL FIELD

The present disclosure relates to a heart-lung machine (HLM) and methods for manufacturing and/or using a heart-lung machine. More particularly, the present disclosure relates to aspects of a controller and/or configuration(s) for a heart-lung machine and methods for manufacturing and/or using a heart-lung machine.

### BACKGROUND

Some medical procedures (e.g., medical procedures which treat cardiac or respiratory disease) may require the use of a heart-lung machine (HLM) that artificially supports cardiac and pulmonary function(s). An HLM may provide temporary and/or short-term support to a patient during a cardiac procedure. In some procedures, the HLM may permit temporarily stopping the patient's heart. An HLM works by removing blood from a patient's body to oxygenate the red blood cells while also removing carbon dioxide. The oxygenated blood is then returned to the patient.

HLMs may include multiple devices that together form a blood recirculation loop between the patient and a blood oxygenator. For example, some HLMs may include a blood reservoir, a blood pump to power blood flow, an oxygenator to oxygenate the blood, a device to filter the blood (which may be included within the oxygenator and/or the reservoir in some systems), one or more sensors positioned at various locations along blood pathways, and one or more control units. It can be appreciated that a blood pathway (e.g., tubing) may extend from the patient to the blood reservoir, then towards a blood pump, then pass through the oxygenator, and close the loop by returning to the patient. Accordingly, the blood pump may assist the heart by pumping blood through the circulation loop, while the oxygenator may assist the lungs by oxygenating blood that is eventually returned to the patient.

When setting up the HLM, measured values from the one or more sensors are available immediately after switching on the HLM. In some systems, the controller may include and/or display one or more alarms corresponding to measured values that are outside of predetermined limits. Alarms related to set-up conditions (as opposed to alarms generated during use) may sometimes be considered as false alarms. False alarms may be problematic in that users may ignore them, and then later overlook or ignore real alarms, thereby compromising patient safety. False alarms and/or the expectation of them may also result in missed steps and/or activations during system set-up. There is an ongoing need for alternative devices, components, configurations, and/or methods of use and/or manufacture of said devices and/or components to improve usability and/or patient safety.

### SUMMARY

In one example, a heart-lung machine (HLM) system may comprise a controller, and a plurality of venous sensors disposed along a venous pathway, the plurality of venous sensors comprising a venous blood gas sensor. Upon activation of the HLM system, the controller is configured to suppress alarms associated with the plurality of venous sensors until blood is detected within the venous pathway by the venous blood gas sensor.

In addition or alternatively to any example described herein, the HLM system may comprise a plurality of arterial sensors disposed along an arterial pathway.

In addition or alternatively to any example described herein, upon activation of the HLM system, the controller is configured to suppress alarms associated with the plurality of arterial sensors until blood is detected within the venous pathway by the venous blood gas sensor.

In addition or alternatively to any example described herein, the HLM system may comprise an oxygenator, a capnograph sensor configured to detect an exhaled carbon dioxide content leaving the oxygenator, and a gas mixture flow sensor configured to detect a flow rate of a compressed air and oxygen gas mixture flowing into the oxygenator.

In addition or alternatively to any example described herein, the controller is configured to automatically activate the capnograph sensor without alarm limits when a case is started within the controller.

In addition or alternatively to any example described herein, the controller is configured to automatically activate alarm limits associated with the capnograph sensor upon detection of blood within the venous pathway by the venous blood gas sensor.

In addition or alternatively to any example described herein, the controller is configured to automatically activate the gas mixture flow sensor and set the flow rate of the compressed air and oxygen gas mixture entering the oxygenator to zero liters per minute when a case is started within the controller.

In addition or alternatively to any example described herein, upon detection of blood within the venous pathway by the venous blood gas sensor, the controller is configured to automatically check if the flow rate of the compressed air and oxygen gas mixture entering the oxygenator is greater than zero liters per minute and to activate a gas flow notification if the flow rate of the compressed air and oxygen gas mixture entering the oxygenator is not greater than zero liters per minute.

In addition or alternatively to any example described herein, upon detection of blood within the venous pathway by the venous blood gas sensor, the controller is configured to automatically set the flow rate of the compressed air and oxygen gas mixture to a predetermined value.

In addition or alternatively to any example described herein, a heart-lung machine (HLM) system may comprise a controller, an oxygenator, a venous pathway configured to transport deoxygenated blood from at least one venous portion of a patient's vasculature to the oxygenator, an arterial pathway configured to transport oxygenated blood from the oxygenator to at least one arterial portion of the patient's vasculature, and a plurality of venous sensors disposed along the venous pathway. The plurality of venous sensors may comprise a venous blood gas sensor. The venous pathway and the arterial pathway may cooperate to bypass blood flow through a patient's heart. The controller may include a bypass timer configured to document an amount of time that blood flow bypasses the patient' s heart while the HLM system is active. The controller may be configured to start the bypass timer upon initial detection of blood within the venous pathway by the venous blood gas sensor.

In addition or alternatively to any example described herein, upon activation of the HLM system, the controller is configured to suppress alarms associated with the plurality of venous sensors until blood is detected within the venous pathway by the venous blood gas sensor.

In addition or alternatively to any example described herein, the HLM system may comprise a capnograph sensor configured to detect an exhaled carbon dioxide content leaving the oxygenator, wherein the controller is configured to automatically activate alarm limits associated with the capnograph sensor upon detection of blood within the venous pathway by the venous blood gas sensor.

In addition or alternatively to any example described herein, the controller is configured to start the bypass timer before the patient's heart is completely bypassed by the HLM system.

In addition or alternatively to any example described herein, the controller is configured to automatically stop the bypass timer when the HLM system is deactivated.

In addition or alternatively to any example described herein, a method of bypassing a patient's heart with a heart-lung machine (HLM) system may comprise activating the HLM system, the HLM system comprising a controller, and a plurality of venous sensors disposed along a venous pathway, the plurality of venous sensors comprising a venous blood gas sensor; coupling a venous pathway of the HLM system to at least one venous portion of a patient's vasculature; coupling an arterial pathway of the HLM system to at least one arterial portion of the patient' s vasculature; and suppressing alarms associated with the plurality of venous sensors until blood is detected within the venous pathway by the venous blood gas sensor.

In addition or alternatively to any example described herein, the method may comprise automatically starting a bypass timer configured to document an amount of time that blood flow bypasses the patient's heart while the HLM system is active upon detection of blood within the venous pathway by the venous blood gas sensor.

In addition or alternatively to any example described herein, the method may comprise automatically activating alarm limits associated with a capnograph sensor configured to detect an exhaled carbon dioxide content leaving an oxygenator of the HLM system upon detection of blood within the venous pathway by the venous blood gas sensor.

In addition or alternatively to any example described herein, the method may comprise automatically activating the capnograph sensor without alarm limits when a case is started within the controller prior to detection of blood within the venous pathway by the venous blood gas sensor.

In addition or alternatively to any example described herein, the method may comprise: when a case is started within the controller prior to detection of blood within the venous pathway by the venous blood gas sensor: automatically activating a gas mixture flow sensor configured to detect a flow rate of a compressed air and oxygen gas mixture flowing into an oxygenator of the HLM system; and setting the flow rate of the compressed air and oxygen gas mixture entering the oxygenator to zero liters per minute.

In addition or alternatively to any example described herein, the method may comprise upon detection of blood within the venous pathway by the venous blood gas sensor, automatically checking if the flow rate of the compressed air and oxygen gas mixture entering the oxygenator is greater than zero liters per minute and activating a gas flow notification if the flow rate of the compressed air and oxygen gas mixture entering the oxygenator is not greater than zero liters per minute.

The above summary of some embodiments, aspects, and/or examples is not intended to describe each disclosed embodiment or every implementation of the present disclosure. The figures and detailed description which follow more particularly exemplify these embodiments.

### BRIEF DESCRIPTION OF THE DRAWINGS

The disclosure may be more completely understood in consideration of the following detailed description in connection with the accompanying drawings, in which:
FIG. 1 schematically illustrates selected aspects of an HLM;
FIG. 2 schematically illustrates selected aspects of an HLM;
FIG. 3 schematically illustrates selected aspects of an HLM;
FIG. 4 schematically illustrates selected aspects of an HLM;
FIG. 5 is a flow chart schematically illustrating selected aspects of a method of bypassing a patient's heart with an HLM; and
FIG. 6 is a flow chart schematically illustrating selected aspects of a method of bypassing a patient's heart with an HLM.

While aspects of the disclosure are amenable to various modifications and alternative forms, specifics thereof have been shown by way of example in the drawings and will be described in detail. It should be understood, however, that the intention is not to limit aspects of the disclosure to the particular embodiments described. On the contrary, the intention is to cover all modifications, equivalents, and alternatives falling within the spirit and scope of the disclosure.

### DETAILED DESCRIPTION

The following description should be read with reference to the drawings, which are not necessarily to scale, wherein like reference numerals indicate like elements throughout the several views. The detailed description and drawings are intended to illustrate example embodiments of the disclosure but not limit the disclosure. Those skilled in the art will recognize that the various elements described and/or shown may be arranged in various combinations and configurations without departing from the scope of the disclosure. However, in the interest of clarity and ease of understanding, every feature and/or element may not be shown in each drawing.

For the following defined terms, these definitions shall be applied, unless a different definition is given in the claims or elsewhere in this specification.

As used herein in association with values (e.g., terms of magnitude, measurement, and/or other degrees of qualitative and/or quantitative observations that are used herein with respect to characteristics (e.g., dimensions, measurements, attributes, components, etc.) and/or ranges thereof, of tangible things (e.g., products, inventory, etc.) and/or intangible things (e.g., data, electronic representations of currency, accounts, information, portions of things (e.g., percentages, fractions), calculations, data models, dynamic system models, algorithms, parameters, etc.), "about" and "approximately" may be used, interchangeably, to refer to a value, configuration, orientation, and/or other characteristic that is equal to (or the same as) the stated value, configuration, orientation, and/or other characteristic or equal to (or the same as) a value, configuration, orientation, and/or other characteristic that is reasonably close to the stated value, configuration, orientation, and/or other characteristic, but that may differ by a reasonably small amount such as will be understood, and readily ascertained, by individuals having ordinary skill in the relevant arts to be attributable to measurement error; differences in measurement and/or manufacturing equipment calibration; human error in reading and/or setting measurements; adjustments made to optimize performance and/or structural parameters in view of other measurements (e.g., measurements associated with other things); particular implementation scenarios; imprecise adjustment and/or manipulation of things, settings, and/or measurements by a person, a computing device, and/or a machine; system tolerances; control loops; machine-learning; foreseeable variations (e.g., statistically insignificant variations, chaotic variations, system and/or model instabilities, etc.); preferences; and/or the like.

The recitation of numerical ranges by endpoints includes all numbers within that range (e.g., 1 to 5 includes 1, 1.5, 2, 2.75, 3, 3.80, 4, and 5). All numeric values are herein assumed to be modified by the term "about", whether or not explicitly indicated. In some instances, the term "about" may include numbers that are rounded to the nearest significant figure.

As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise.

It is noted that references in the specification to "an embodiment", "some embodiments", "other embodiments", etc., indicate that the embodiment described may include one or more particular features, structures, and/or characteristics. However, such recitations do not necessarily mean that all embodiments include the particular features, structures, and/or characteristics. Additionally, when particular features, structures, and/or characteristics are described in connection with one embodiment, it should be understood that such features, structures, and/or characteristics may also be used in connection with other embodiments whether or not explicitly described unless clearly stated to the contrary.

Additionally, it should be noted that in any given figure, some features may not be shown, or may be shown schematically, for clarity and/or simplicity. Additional details regarding some components and/or method steps may be illustrated in other figures in greater detail. The devices and/or methods disclosed herein may provide a number of desirable features and benefits as described in more detail below.

In a normal heart, blood circulates via a closed path whereby deoxygenated (venous) blood enters the right atrium via both the superior vena cava and inferior vena cava. The venous blood then passes through the right ventricle and is pumped via the pulmonary artery to the lungs, where it absorbs oxygen and releases carbon dioxide. After absorbing oxygen and releasing carbon dioxide in the lungs, the blood becomes oxygenated arterial blood. The oxygenated blood is then returned via the pulmonary veins to the left atrium and is passed to the left ventricle. The oxygenated arterial blood is then pumped through the aorta and eventually throughout the body.

It can be appreciated that if the lungs are incapable of sufficiently oxygenating blood and/or removing carbon dioxide, an oxygenator located outside the body may be used to oxygenate the blood and/or remove carbon dioxide. As discussed above, an HLM may be utilized to support patients while medical treatments (e.g., heart surgery) are performed to treat an underlying illness. When supported via extracorporeal perfusion, oxygenation of the patient's blood and removal of carbon dioxide may occur outside the body.

Extracorporeal perfusion is generally performed using an HLM, wherein a system comprising an HLM may be referred to as a "circuit." The circuit may include at least one blood pathway exterior of the patient, such as one or more tubing pathways designed to transfer blood from a patient's body to the oxygenator and back into the patient. As described above, the oxygenator may add oxygen to the blood while also removing carbon dioxide (e.g., the oxygenator performs the function of a healthy lung).

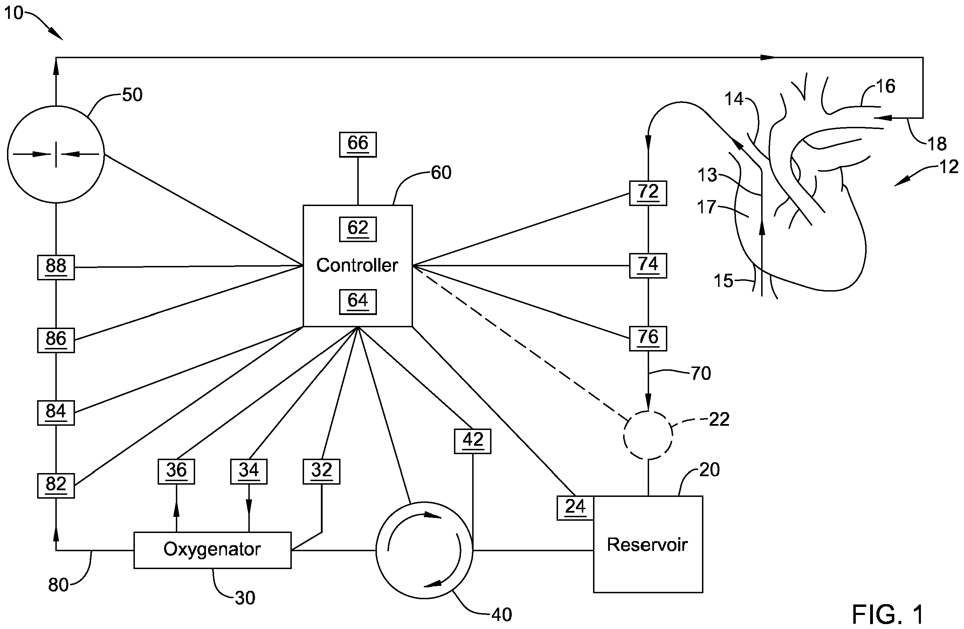

FIG. 1 schematically illustrates selected aspects of a heart-lung machine (HLM) system 10. In at least some embodiments, the HLM system 10 may be configured to bypass a patient's heart 12 during a cardiac procedure. In some embodiments, the HLM system 10 may comprise a blood reservoir 20, an oxygenator 30, a pump 40, a clamp 50, and a controller 60. Other configurations are also contemplated.

The HLM system 10 may comprise a venous pathway 70 fluidly couplable to a patient and/or a patient's vasculature (e.g., the heart, one or more veins, etc.) for transporting deoxygenated fluid (e.g., blood) from the patient's vasculature toward, to, and/or within the HLM system 10 and/or the oxygenator 30. In at least some embodiments, the venous pathway 70 may comprise tubing and/or a plurality of tubing portions extending between various components and/or elements of the venous pathway 70 and/or extending between various components and/or elements disposed along the venous pathway 70. In some embodiments, the tubing and/or the plurality of tubing portions may comprise polymeric material(s) and/or biocompatible material(s). In some embodiments, the HLM system may further comprise a venous cannula or cannulas 13 configured for insertion into and/or configured to engage at least one venous portion of the patient's vasculature. In one example, the venous cannula or cannulas 13 may be configured for insertion into and/or may be configured to engage a superior vena cava (SVC) 14 and/or an inferior vena cava (IVC) 15 of the patient's vasculature adjacent to and/or near the patient's heart 12. In another example, the venous cannula or cannulas 13 may be configured for insertion into and/or may be configured to engage a right atrium 17 of the patient's heart 12. In some embodiments, the venous pathway 70 may be configured to transport deoxygenated fluid (e.g., blood) from at least one venous portion of a patient's vasculature to the HLM system 10 and/or the oxygenator 30. Other configurations are also contemplated.

The HLM system 10 may comprise a plurality of venous sensors disposed along, coupled to, and/or in communication with the venous pathway 70. In some embodiments, the plurality of venous sensors may be in fluid communication with the venous pathway 70 and/or fluid (e.g., blood, priming fluid, etc.) disposed and/or flowing therein. In some embodiments, the plurality of venous sensors may be in communication (e.g., electronic communication, wired communication, wireless communication, etc.) with the controller 60. In some embodiments, the plurality of venous sensors may include, but are not limited to, a venous blood gas sensor 72, a venous flow sensor 74, and/or a venous pressure sensor 76. In some embodiments, the HLM system and/or the plurality of venous sensors may comprise one or more sensors for monitoring flow rates, pressures, temperatures, bubbles, oxygen saturation, carbon dioxide content, blood gases, or other fluid (e.g., blood, priming fluid, etc.) parameters. Additionally, in some embodiments, a single sensor may be configured to sense multiple fluid (e.g., blood, priming fluid, etc.) parameters including flow rate, pressures, temperatures, bubbles, oxygen saturation, carbon dioxide content, blood gases, etc. Other configurations are also contemplated. It is noted that the exact order and/or placement of the plurality of venous sensors disposed along, coupled to, and/or in communication with the venous pathway 70 may vary from that shown in the figures, unless expressly stated otherwise.

The HLM system 10 may comprise an arterial pathway 80 fluidly couplable to the patient and/or the patient's vasculature (e.g., the heart, one or more arteries, etc.) for transporting oxygenated fluid (e.g., blood, priming fluid, etc.) within the HLM system 10 and/or from the HLM system 10 and/or the oxygenator 30 toward and/or to the patient's vasculature. In at least some embodiments, the arterial pathway 80 may comprise tubing and/or a plurality of tubing portions extending between various components and/or elements of the arterial pathway 80 and/or extending between various components and/or elements disposed along the arterial pathway 80. In some embodiments, the tubing and/or the plurality of tubing portions may comprise polymeric material(s) and/or biocompatible material(s). In some embodiments, the HLM system may further comprise an arterial cannula or cannulas 18 configured for insertion into and/or configured to engage at least one arterial portion of the patient's vasculature. In one example, the arterial cannula or cannulas 18 may be configured for insertion into and/or may be configured to engage an aorta 16 of the patient's vasculature adjacent to and/or near the patient's heart 12. In some embodiments, the arterial pathway 80 may be configured to transport oxygenated fluid (e.g., blood, priming fluid, etc.) from the HLM system 10 and/or the oxygenator 30 to at least one arterial portion of the patient's vasculature. Other configurations are also contemplated.

In some embodiments, the blood reservoir 20 may be configured and/or adapted to hold fluid (e.g., blood, priming fluid, etc.) which is gravity fed from the at least one venous portion of the patient's vasculature. Accordingly, the blood reservoir 20 may be disposed at a lower elevation or location compared to the patient and/or the patient's heart 12. In some embodiments and/or under some conditions, gravity alone may be unable or insufficient to supply an adequate volume of fluid (e.g., blood, priming fluid, etc.) to the blood reservoir 20 to support the procedure. As such, in some embodiments, the HLM system 10 may optionally comprise a second pump 22 disposed along, coupled to, and/or in communication with the venous pathway 70 upstream of the blood reservoir 20. In some embodiments, the second pump 22 may be in fluid communication with the venous pathway 70 and/or fluid (e.g., blood, priming fluid, etc.) disposed and/or flowing therein. In some embodiments, the second pump 22 may be disposed completely external to the venous pathway 70 and/or may be devoid of fluid communication with the fluid (e.g., blood, priming fluid, etc.) flowing within the venous pathway 70.

In some embodiments, the second pump 22 may be in communication (e.g., electronic communication, wired communication, wireless communication, etc.) with the controller 60. In some embodiments, the second pump 22 may be configured to generate fluid flow and/or pressure along and/or within the venous pathway 70, thereby enhancing the flow of fluid (e.g., blood, priming fluid, etc.) within the venous pathway 70 and/or to the blood reservoir 20. In some embodiments, the second pump 22 may be a roller pump. In some embodiments, the second pump 22 may be a centrifugal pump. Other configurations and/or pump types are also contemplated.

In some embodiments, the HLM system 10 and/or the blood reservoir 20 may comprise a blood level sensor 24 coupled to and/or in communication with the blood reservoir 20. In some embodiments, the blood level sensor 24 may be integrated with the blood reservoir 20. In some embodiments, the blood level sensor 24 may be configured to detect and/or monitor a level of fluid (e.g., blood, priming fluid, etc.) within the blood reservoir 20. In some embodiments, the blood level sensor 24 may be configured to detect and/or monitor whether the level of fluid (e.g., blood, priming fluid, etc.) within the blood reservoir 20 falls below a predetermined minimum level. In some embodiments, the blood level sensor 24 may be configured to detect and/or monitor whether the level of fluid (e.g., blood, priming fluid, etc.) within the blood reservoir 20 exceeds a predetermined maximum level. In some embodiments, the blood level sensor 24 may be configured to detect and/or monitor the level of fluid (e.g., blood, priming fluid, etc.) within the blood reservoir 20 at any time and/or at all times. For example, the blood level sensor 24 may be configured to detect and/or measure the level of fluid (e.g., blood, priming fluid, etc.) within the blood reservoir 20 continuously and/or at any level. The blood level sensor 24 may be in communication (e.g., electronic communication, wired communication, wireless communication, etc.) with the controller 60. Other configurations are also contemplated.

As discussed herein, the HLM system 10 may comprise the pump 40. In some embodiments, the pump 40 may be disposed along, coupled to, and/or in communication with the venous pathway 70 downstream of the blood reservoir 20 and/or upstream of the oxygenator 30. In some embodiments, the pump 40 may be in fluid communication with the venous pathway 70 and/or fluid (e.g., blood, priming fluid, etc.) disposed and/or flowing therein. In some embodiments, the pump 40 may be disposed completely external to the venous pathway 70 and/or may be devoid of fluid communication with the fluid (e.g., blood, priming fluid, etc.) flowing within the venous pathway 70.

In some embodiments, the pump 40 may be in communication (e.g., electronic communication, wired communication, wireless communication, etc.) with the controller 60. In some embodiments, the controller 60 may be configured to generate fluid flow and/or pressure along and/or within the venous pathway 70 using the pump 40, thereby causing the flow of fluid (e.g., blood, priming fluid, etc.) within the venous pathway 70 and/or from the blood reservoir 20 to the oxygenator 30. In some embodiments, the pump 40 may be a roller pump. In some embodiments, the pump 40 may be a centrifugal pump. Other configurations and/or pump types are also contemplated. It can be appreciated that the function of the pump 40 is to generate blood flow within the HLM system (e.g., circulate blood from the patient to the oxygenator and back to the patient) and to also generate blood pressure and facilitate blood flow within the patient's vasculature.

In some embodiments, the controller 60 may be configured to generate fluid flow and/or pressure along and/or within the arterial pathway 80 and/or at least a portion of the venous pathway 70 using the pump 40, thereby causing the flow of fluid (e.g., blood, priming fluid, etc.) within the venous pathway 70 and/or the arterial pathway 80 downstream of and/or from the blood reservoir 20, through the oxygenator 30, and/or to the arterial portion of the patient's vasculature. Other configurations are also contemplated.

In some embodiments, the controller 60 may be configured to utilize feedback and/or signals from the blood level sensor 24 to control and/or modulate the pump 40 and/or the second pump 22 (where present) to control and/or manipulate the level of fluid (e.g., blood, priming fluid, etc.) within the blood reservoir 20. For example, if the blood level sensor 24 indicates that the level of fluid (e.g., blood, priming fluid, etc.) within the blood reservoir 20 is at and/or is approaching the predetermined minimum level, the controller 60 may be configured to change parameters associated with the pump 40 and/or the second pump 22 to increase the flow of fluid (e.g., blood, priming fluid, etc.) into the blood reservoir 20 and/or to decrease the flow of fluid (e.g., blood, priming fluid, etc.) out of the blood reservoir 20 so as to increase the level of fluid (e.g., blood, priming fluid, etc.) within the blood reservoir 20. In another example, if the blood level sensor 24 indicates that the level of fluid (e.g., blood, priming fluid, etc.) within the blood reservoir 20 is at and/or is approaching the predetermined maximum level, the controller 60 may be configured to change parameters associated with the pump 40 and/or the second pump 22 to decrease the flow of fluid (e.g., blood, priming fluid, etc.) into the blood reservoir 20 and/or to increase the flow of fluid (e.g., blood, priming fluid, etc.) out of the blood reservoir 20 so as to decrease the level of fluid (e.g., blood, priming fluid, etc.) within the blood reservoir 20. Other configurations are also contemplated.

In some embodiments, the HLM system 10 and/or the pump 40 may comprise a pump inlet pressure sensor 42 coupled to and/or in communication with the pump 40 and/or the venous pathway 70 at and/or proximate a fluid inlet into the pump 40. In some embodiments, the pump inlet pressure sensor 42 may be integrated with the pump 40. In some embodiments, the pump inlet pressure sensor 42 may be configured to detect and/or measure a fluid pressure of the fluid (e.g., blood, priming fluid, etc.) flowing within the venous pathway 70 as it enters the pump 40. The pump inlet pressure sensor 42 may be in communication (e.g., electronic communication, wired communication, wireless communication, etc.) with the controller 60. In some embodiments, the pump inlet pressure sensor 42 may be used by the controller 60 to modulate and/or control the pump 40 (e.g., pump speed, voltage and/or current supplied, etc.) to generate the correct and/or desired flow rate and/or pressure downstream of the pump 40. For example, the controller 60 may vary a pump speed of the pump 40 based on the fluid pressure detected by the pump inlet pressure sensor 42. Other configurations are also contemplated.

In some embodiments, the HLM system 10 may comprise the oxygenator 30. In some embodiments, the oxygenator 30 may include a housing having multiple chambers or pathways separated by a semi-permeable membrane, whereby the patient's blood may flow through one chamber or pathway, while a compressed air and oxygen gas mixture flows through another chamber or pathway. The semi-permeable membrane may include multiple microporous hollow fibers, each fiber having a lumen extending therethrough through which the compressed air and oxygen gas mixture flows. Gas exchange may occur via diffusion of the gases across the multiple microporous hollow fibers, whereby oxygen moves from the inside of the multiple microporous hollow fibers into the blood while carbon dioxide diffuses from the blood into the interior of the multiple microporous hollow fibers, where it is swept away by the compressed air and oxygen gas mixture flowing through the multiple microporous hollow fibers. This gas exchange allows for oxygenation of venous blood and removal of carbon dioxide.

The oxygenator 30 may be disposed downstream of the blood reservoir 20. In some embodiments, the oxygenator 30 may be disposed downstream of the pump 40. In some embodiments, the blood reservoir 20 and the oxygenator 30 may be integrated and/or combined into a single unit. In some embodiments, the blood reservoir 20 and the oxygenator 30 may be separate units and/or structures that are fluidly coupled to together. Other configurations are also contemplated

In some embodiments, the HLM system 10 and/or the oxygenator 30 may comprise a heat exchanger (not explicitly shown) configured to heat and/or cool blood flowing therethrough prior to returning the blood to the arterial portion of the patient's vasculature. In some embodiments, the heat exchanger may be integrated with the oxygenator 30. In some embodiments, the oxygenator 30 and/or the heat exchanger may be coupled to and/or may be in fluid communication with the blood reservoir 20. In some embodiments, the blood reservoir 20, the oxygenator 30, and/or the heat exchanger may be integrated and/or combined into a single unit. In some embodiments, the oxygenator 30 and/or the heat exchanger may be integrated and/or combined into a single unit, while the blood reservoir 20 is a separate unit and/or structure that is fluidly coupled to the single unit comprising the oxygenator 30 and the heat exchanger. Other configurations are also contemplated.

In some embodiments, the HLM system 10 and/or the oxygenator 30 may comprise a pre-oxygenator pressure sensor 32 coupled to and/or in communication with the oxygenator 30 and/or the venous pathway 70 at and/or proximate a fluid inlet into the oxygenator 30. In some embodiments, the pre-oxygenator pressure sensor 32 may be disposed upstream of the oxygenator 30 and downstream of the blood reservoir 20 and/or the pump 40. In some embodiments, the pre-oxygenator pressure sensor 32 may be integrated with the oxygenator 30. In some embodiments, the pre-oxygenator pressure sensor 32 may be configured to detect and/or measure a fluid pressure of the fluid (e.g., blood, priming fluid, etc.) flowing within the venous pathway 70 as it enters the oxygenator 30. The pre-oxygenator pressure sensor 32 may be in communication (e.g., electronic communication, wired communication, wireless communication, etc.) with the controller 60. In some embodiments, the pre-oxygenator pressure sensor 32 may be used by the controller 60 to modulate and/or control the pump 40 (e.g., pump speed, voltage and/or current supplied, etc.) to generate the correct and/or desired flow rate and/or pressure entering the oxygenator 30. For example, the controller 60 may vary a pump speed of the pump 40 based on the fluid pressure detected by the pre-oxygenator pressure sensor 32. Other configurations are also contemplated.

During operation of the HLM system 10 and/or the oxygenator 30, the compressed air and oxygen gas mixture may flow into the oxygenator 30 through an air inlet and out of the oxygenator 30 through an air outlet. In some embodiments, the HLM system 10 and/or the oxygenator 30 may comprise a gas mixture flow sensor 34 and/or a capnograph sensor 36. The gas mixture flow sensor 34 may be coupled to and/or in communication with the oxygenator 30 at and/or proximate the air inlet into the oxygenator 30. In some embodiments, the gas mixture flow sensor 34 may be integrated with the oxygenator 30. In some embodiments, the gas mixture flow sensor 34 may be configured to detect and/or measure a flow rate of the compressed air and oxygen gas mixture flowing into the oxygenator 30. The gas mixture flow sensor 34 may be in communication (e.g., electronic communication, wired communication, wireless communication, etc.) with the controller 60. In some embodiments, the gas mixture flow sensor 34 may be used by the controller 60 to modulate and/or control the flow and/or mixture of the compressed air and oxygen gas mixture. Other configurations are also contemplated. The capnograph sensor 36 may be coupled to and/or in communication with the oxygenator 30 at and/or proximate the air outlet from the oxygenator 30. In some embodiments, the capnograph sensor 36 may be integrated with the oxygenator 30. In some embodiments, the capnograph sensor 36 may be configured to detect and/or measure an exhaled carbon dioxide (ExCO2) content in the compressed air and oxygen gas mixture leaving the oxygenator 30. The exhaled carbon dioxide content may be indicative of and/or may approximately correspond to the carbon dioxide content of the exhaled air of a human being and may be related to and/or indicative of proper function of the oxygenator 30. The capnograph sensor 36 may be in communication (e.g., electronic communication, wired communication, wireless communication, etc.) with the controller 60. Other configurations are also contemplated.

The arterial pathway 80 may extend downstream from the oxygenator 30 and/or may extend from the oxygenator 30 toward and/or to the arterial cannula or cannulas 18 configured for insertion into and/or configured to engage at least one arterial portion of the patient's vasculature. The HLM system 10 may comprise a plurality of arterial sensors disposed along, coupled to, and/or in communication with the arterial pathway 80. In some embodiments, the plurality of arterial sensors may be in fluid communication with the arterial pathway 80 and/or fluid (e.g., blood, priming fluid, etc.) disposed and/or flowing therein. In some embodiments, the plurality of arterial sensors may be in communication (e.g., electronic communication, wired communication, wireless communication, etc.) with the controller 60. In some embodiments, the plurality of arterial sensors may include, but are not limited to, an arterial blood gas sensor 82, an arterial flow sensor 84, an arterial pressure sensor 86, and/or an arterial bubble sensor 88. In some embodiments, the HLM system 10 and/or the plurality of arterial sensors may comprise one or more sensors for monitoring flow rates, pressures, temperatures, bubbles, oxygen saturation, carbon dioxide content, blood gases, or other fluid (e.g., blood, priming fluid, etc.) parameters. Additionally, in some embodiments, a single sensor may be configured to sense multiple fluid (e.g., blood, priming fluid, etc.) parameters including flow rate, pressures, temperatures, bubbles, oxygen saturation, carbon dioxide content, blood gases, etc. Other configurations are also contemplated. It is noted that the exact order and/or placement of the plurality of arterial sensors disposed along, coupled to, and/or in communication with the arterial pathway 80 may vary from that shown in the figures, unless expressly stated otherwise.

In some embodiments, the HLM system 10 may comprise the clamp 50. In some embodiments, the clamp 50 may be disposed along, coupled to, and/or in communication with the arterial pathway 80 (e.g., the tubing and/or the plurality of tubing portions thereof) downstream of the oxygenator 30. It is noted that the exact placement of the clamp 50 disposed along, coupled to, and/or in communication with the arterial pathway 80 may vary from that shown in the figures, unless expressly stated otherwise. In some embodiments, the clamp 50 may be in fluid communication with the arterial pathway 80 and/or fluid (e.g., blood, priming fluid, etc.) disposed and/or flowing therein. In some embodiments, the clamp 50 may be disposed completely external to the arterial pathway 80 (e.g., the tubing and/or the plurality of tubing portions thereof) and/or may be devoid of fluid communication with the fluid (e.g., blood, priming fluid, etc.) flowing within the arterial pathway 80 (e.g., the tubing and/or the plurality of tubing portions thereof) to avoid contamination of the fluid (e.g., blood, priming fluid, etc.).

In some embodiments, the clamp 50 may be an electronic remote clamp (ERC). In some embodiments, the clamp 50 may be in communication (e.g., electronic communication, wired communication, wireless communication, etc.) with the controller 60. In some embodiments, the controller 60 may be configured to control and/or regulate fluid flow along and/or within the arterial pathway 80 (e.g., the tubing and/or the plurality of tubing portions thereof) using the clamp 50, and signals from the plurality of venous sensors and/or signals from the plurality of arterial sensors. For example, the lumen of the tubing may have a cross-sectional area which, along with the velocity of the fluid (e.g., blood, priming fluid, etc.) flowing through the tubing, defines the volume of fluid (e.g., blood, priming fluid, etc.) which may pass through the tubing over a given time period. In some embodiments, the controller 60 may be configured to actuate the clamp 50 to change the cross-sectional area of the lumen of the arterial pathway 80 (e.g., the tubing and/or the plurality of tubing portions thereof) to increase and/or reduce the cross-sectional area of the lumen of the arterial pathway 80 and thereby change and/or affect fluid flow within the arterial pathway 80 (and by extension, the venous pathway 70 and the HLM system 10 as a whole). In some embodiments, the clamp 50 may be configured to and/or may be capable of completely closing off the lumen of the arterial pathway 80, thereby stopping fluid flow within the arterial pathway 80 and/or the HLM system 10. In some embodiments, the clamp 50 may be a manually operated and/or actuated clamp and control of the clamp 50 may be performed manually by a user or clinician. Other configurations are also contemplated.

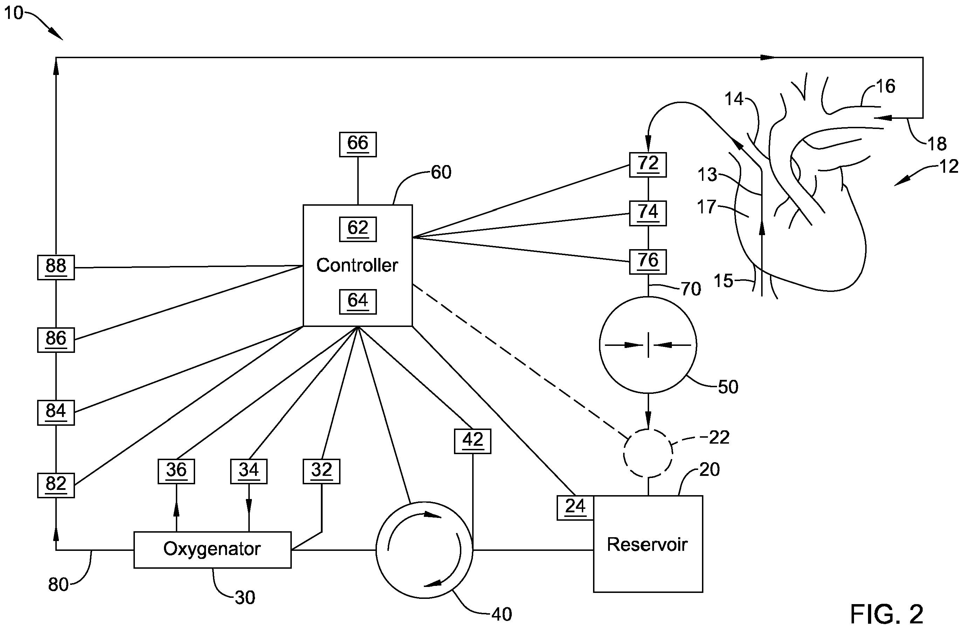

In some alternative configurations, the clamp 50 may be disposed along, coupled to, and/or in communication with the venous pathway 70 (e.g., the tubing and/or the plurality of tubing portions thereof) upstream of the blood reservoir 20 and/or the second pump 22, as shown schematically in FIG. 2. It is noted that the exact placement of the clamp 50 disposed along, coupled to, and/or in communication with the venous pathway 70 may vary from that shown in the figures, unless expressly stated otherwise. In some embodiments, the clamp 50 may be in fluid communication with the venous pathway 70 and/or fluid (e.g., blood, priming fluid, etc.) disposed and/or flowing therein. In some embodiments, the clamp 50 may be disposed completely external to the venous pathway 70 (e.g., the tubing and/or the plurality of tubing portions thereof) and/or may be devoid of fluid communication with the fluid (e.g., blood, priming fluid, etc.) flowing within the venous pathway 70 (e.g., the tubing and/or the plurality of tubing portions thereof) to avoid contamination of the fluid (e.g., blood, priming fluid, etc.). In some embodiments, the controller 60 may be configured to control and/or regulate fluid flow along and/or within the venous pathway 70 (e.g., the tubing and/or the plurality of tubing portions thereof) using the clamp 50, and signals from the plurality of venous sensors and/or signals from the plurality of arterial sensors. For example, the controller 60 may be configured to actuate the clamp 50 to change a cross-sectional area of a lumen of the venous pathway 70 (e.g., the tubing and/or the plurality of tubing portions thereof) to increase and/or reduce the cross-sectional area of the lumen of the venous pathway 70 and thereby change and/or affect fluid flow within the venous pathway 70 (and by extension, the arterial pathway 80 and the HLM system 10 as a whole). Other configurations are also contemplated.

In some further alternative configurations, the HLM system 10 may comprise a plurality of clamps disposed along, coupled to, and/or in communication with the venous pathway 70 (e.g., the tubing and/or the plurality of tubing portions thereof) and/or the arterial pathway 80 (e.g., the tubing and/or the plurality of tubing portions thereof). The plurality of clamps may be in communication (e.g., electronic communication, wired communication, wireless communication, etc.) with the controller 60, and the controller 60 may be configured to control and/or regulate fluid flow along and/or within the venous pathway 70 (e.g., the tubing and/or the plurality of tubing portions thereof) and/or the arterial pathway 80 (e.g., the tubing and/or the plurality of tubing portions thereof) using the plurality of clamps.

The clamp 50 is described above with respect to the clamp 50 being disposed external to the arterial pathway 80 (e.g., the tubing and/or the plurality of tubing portions thereof) and/or the venous pathway 70 (e.g., the tubing and/or the plurality of tubing portions thereof). In other examples, wherein the clamp 50 is in fluid communication with the arterial pathway 80 (e.g., the tubing and/or the plurality of tubing portions thereof) and/or the venous pathway 70 (e.g., the tubing and/or the plurality of tubing portions thereof), and/or the lumen(s) thereof, the clamp 50 may include one or more components (e.g., a ball valve, an iris, etc.) configured to adjust a cross-sectional area of a lumen within and/or passing through the clamp 50. In some embodiments, the clamp 50 may be configured such that a first section of tubing may be coupled with and/or inserted into an inlet of the clamp 50 and a second section of tubing may be coupled with and/or inserted into an outlet of the clamp 50. Accordingly, the fluid (e.g., blood, priming fluid, etc.) may flow through the first section of tubing into the clamp 50, through a valve located in the clamp 50 (e.g., through the one or more components of the clamp 50 configured to adjust the cross-sectional area of the lumen within and/or passing through the clamp 50), and exit the clamp 50 via an outlet of the clamp 50 and into the second section of tubing. Other configurations are also contemplated.

The HLM system 10 may comprise the controller 60. In at least some embodiments, the controller 60 may comprise a visual display 62 and/or one or more control devices 64 (e.g., knobs, buttons, switches, etc.). In some embodiments, the visual display 62 may be separate and/or spaced apart from the controller 60. In some embodiments, the visual display 62 may be integrated with the controller 60. In some embodiments, the one or more control devices 64 may be separate and/or spaced apart from the controller 60. In some embodiments, the one or more control devices 64 may be integrated with the controller 60. In some embodiments, the controller 60 may comprise a touch screen display. In some embodiments, the visual display 62 and/or the one or more controller devices 64 may be and/or may be integrated with the touch screen display. Other configurations are also contemplated.

In some embodiments, the controller 60 may comprise a processor, memory, an input/output (I/O) unit, etc. In some embodiments, the processor may comprise a single processor or more than one processor working individually or with one another. The processor may be configured to execute instructions, including instructions that may be loaded into the memory. Some example processor components may include, but are not limited to, microprocessors, microcontrollers, multi-core processors, graphical processing units, digital signal processors, application specific integrated circuits (ASICs), field programmable gate arrays (FPGAs), discrete circuitry, and/or other suitable types of data processing devices.

In some embodiments, the memory of the controller 60 may include a single memory component or more than one memory component each working individually or with one another. Example types of memory may include random access memory (RAM), EEPROM, FLASH, suitable volatile storage devices, suitable non-volatile storage devices, persistent memory (e.g., read only memory (ROM), hard drive, Flash memory, optical disc memory, and/or other suitable persistent memory) and/or other suitable types of memory. In some embodiments, the memory may be or may include a non-transitory computer readable medium.

The I/O unit of the controller 60 may include a single I/O component or more than one I/O component each working individually or with one another. Some example I/O units may be any type of communication port configured to communicate with other components of the HLM system 10. Some example types of I/O units may include wired ports, wireless ports, radio frequency (RF) ports, Low-Energy Bluetooth ports, Bluetooth ports, Near-Field Communication (NFC) ports, HDMI ports, Wi-Fi ports, Ethernet ports, VGA ports, serial ports, parallel ports, component video ports, S-video ports, composite audio/video ports, DVI ports, USB ports, optical ports, and/or other suitable ports.

As discussed herein, the controller 60 may be in communication with various components of the HLM system 10. In some embodiments, the controller 60 may be integrated into a console or workstation of the HLM system 10. In some embodiments, the various components of the HLM system 10 may together form a closed-loop system capable of automatically or manually regulating the flow rate of fluid (e.g., blood, priming fluid, etc.) within the HLM system 10, the venous pathway 70, and/or the arterial pathway 80. In some examples, the flow rate of fluid (e.g., blood, priming fluid, etc.) within the HLM system 10, the venous pathway 70, and/or the arterial pathway 80 may be from 0-8 liters/minute at a pressure between -200 mmHg and +800 mmHg. Other configurations are also contemplated.

In one example, the plurality of venous sensors and/or the plurality of arterial sensors may be configured to sense a first parameter (e.g., flow rate, pressure, etc.) of fluid (e.g., blood, priming fluid, etc.) passing through the HLM system 10, the venous pathway 70, and/or the arterial pathway 80. Additionally, the plurality of venous sensors and/or the plurality of arterial sensors may be configured to transmit a signal corresponding to the sensed parameter (e.g., flow rate, pressure, etc.) to the controller 60. In some embodiments, the controller 60 may be configured to receive the signal (corresponding to the sensed flow rate and/or pressure of the fluid (e.g., blood, priming fluid, etc.) within the HLM system 10, the venous pathway 70, and/or the arterial pathway 80) transmitted by the plurality of venous sensors and/or the plurality of arterial sensors. In some embodiments, the controller 60 may be configured to compare the signal received from the plurality of venous sensors and/or the plurality of arterial sensors to a parameter (e.g., flow rate, pressure, etc.) set point input by a clinician into the controller 60.

In some embodiments, the venous blood gas sensor 72 may be capable of and/or may be configured to distinguish between an empty tube, blood, priming fluid, and other fluids. In some embodiments, the venous blood gas sensor 72 may be capable of and/or may be configured to distinguish between a lumen of the venous pathway 70 being empty and/or devoid of fluid (e.g., blood, priming fluid, etc.) disposed therein and the venous pathway 70 having fluid (e.g., blood, priming fluid, etc.) disposed therein. In some embodiments, the venous blood gas sensor 72 may be capable of and/or may be configured to distinguish between different types of fluids disposed within the venous pathway 70.

In some embodiments, upon activation of the HLM system 10, the controller 60 may be configured to suppress alarms associated with the plurality of venous sensors until fluid (e.g., blood, priming fluid, etc.) or a particular type of fluid, such as blood, is detected within the venous pathway 70 by the venous blood gas sensor 72. In some embodiments, upon activation of the HLM system 10, the controller 60 may be configured to suppress alarms associated with the plurality of arterial sensors until fluid (e.g., blood, priming fluid, etc.) or a particular type of fluid, such as blood, is detected within the venous pathway 70 by the venous blood gas sensor 72.

In some embodiments, the controller 60 may be configured to automatically activate the blood level sensor 24 associated with the blood reservoir 20 upon detection of fluid (e.g., blood, priming fluid, etc.) or a particular type of fluid, such as blood, within the venous pathway 70 by the venous blood gas sensor 72. In some embodiments, the controller 60 may be configured to automatically activate the arterial bubble sensor 88 upon detection of fluid (e.g., blood, priming fluid, etc.) or a particular type of fluid, such as blood, within the venous pathway 70 by the venous blood gas sensor 72.

In some embodiments, the controller 60 may be configured to automatically activate the capnograph sensor 36 associated with the oxygenator 30 without alarm limits when a case is started within the controller 60. In some embodiments, the controller 60 may be configured to automatically activate alarm limits associated with the capnograph sensor 36 upon detection of fluid (e.g., blood, priming fluid, etc.) or a particular type of fluid, such as blood, within the venous pathway 70 by the venous blood gas sensor 72.

In some embodiments, the controller 60 may be configured to automatically activate the gas mixture flow sensor 34 and set the flow rate of the compressed air and oxygen gas mixture entering the oxygenator 30 to zero liters per minute when a case is started within the controller 60. In some embodiments, upon detection of fluid (e.g., blood, priming fluid, etc.) or a particular type of fluid, such as blood, within the venous pathway 70 by the venous blood gas sensor 72, the controller 60 may be configured to automatically check if the flow rate of the compressed air and oxygen gas mixture entering the oxygenator 30 is greater than zero liters per minute and to activate a gas flow notification if the flow rate of the compressed air and oxygen gas mixture entering the oxygenator 30 is not greater than zero liters per minute. In some embodiments, upon detection of fluid (e.g., blood, priming fluid, etc.) or a particular type of fluid, such as blood, within the venous pathway 70 by the venous blood gas sensor 72, the controller 60 may be configured to automatically set the flow rate of the compressed air and oxygen gas mixture to a predetermined value and/or to a value previously set by the user.

In some embodiments, the controller 60 may include a bypass timer 66 configured to document, record, track, monitor, etc. an amount of time that blood flow bypasses the patient's heart 12 while the HLM system 10 is active. In some embodiments, the bypass timer 66 may be separate and/or spaced apart from the controller 60. In some embodiments, the bypass timer 66 may be integrated with the controller 60. Other configurations are also contemplated. In some embodiments, the bypass timer 66 may be started and/or activated manually by user input. In some embodiments, the HLM system 10 may be active for a length of time before the aorta 16 is clamped off to functionally remove the patient's heart 12 from the patient's vasculature during a cardiac procedure. For example, the HLM system 10 may be active and/or functioning in conjunction with the patient's heart 12 for the length of time before the aorta 16 is clamped off.

In some embodiments, the controller 60 may be configured to start and/or activate the bypass timer 66 when the HLM system 10 is started and/or activated. In some embodiments, the controller 60 may be configured to start and/or activate the bypass timer 66 upon initial detection of fluid (e.g., blood, priming fluid, etc.) or a particular type of fluid, such as blood, within the venous pathway 70 by the venous blood gas sensor 72. In some embodiments, the controller 60 may be configured to automatically start and/or activate the bypass timer 66 upon initial detection of fluid (e.g., blood, priming fluid, etc.) or a particular type of fluid, such as blood, within the venous pathway 70 by the venous blood gas sensor 72. In some embodiments, the controller 60 may be configured to start and/or activate the bypass timer 66 before the aorta 16 is clamped off and/or before the patient's heart 12 is completely bypassed by the HLM system 10. In some embodiments, the aorta 16 may be clamped off at a time after the bypass timer 66 has been started and/or activated (e.g., the HLM system 10 may be activated and/or running for a length of time before the aorta 16 is clamped off). In some embodiments, the length of time before the aorta 16 is clamped off may be several seconds, several minutes, an hour, or more after the bypass timer 66 has been started and/or activated. In some alternative embodiments, the bypass timer 66 may be started and/or activated when the aorta 16 is clamped off.

In some embodiments, the bypass timer 66 may be stopped and/or deactivated manually by user input. The bypass timer 66 may be stopped and/or deactivated, for example, when the patient is taken off bypass and/or when the HLM system 10 is deactivated. In at least some embodiments, the controller 60 may be configured to automatically stop and/or deactivate the bypass timer 66 when the HLM system 10 is deactivated. In some embodiments, the controller 60 may be configured to start the bypass timer 66 upon initial detection of fluid (e.g., blood, priming fluid, etc.) or a particular type of fluid, such as blood, within the venous pathway 70 by the venous blood gas sensor 72.

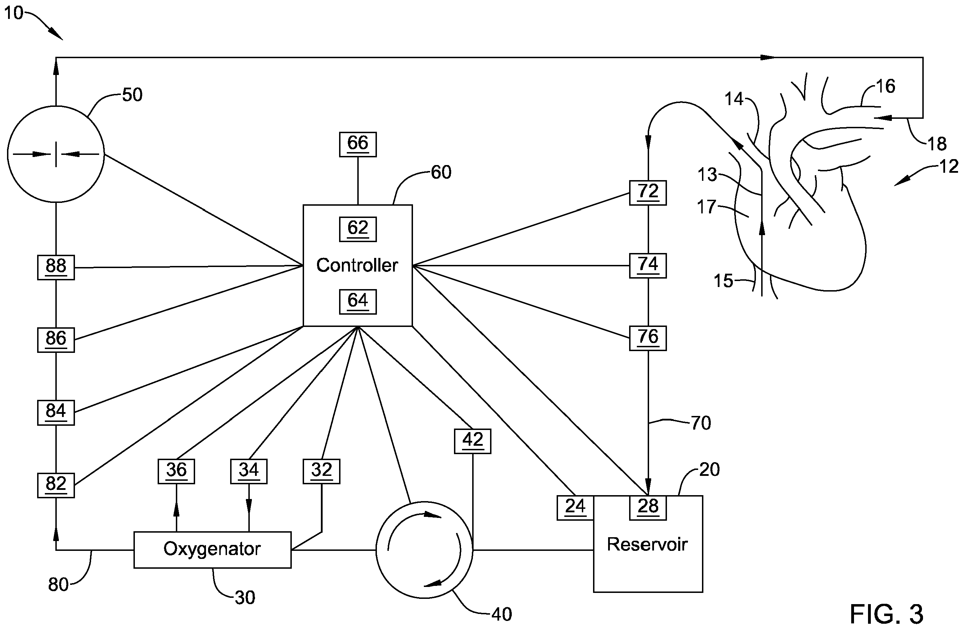

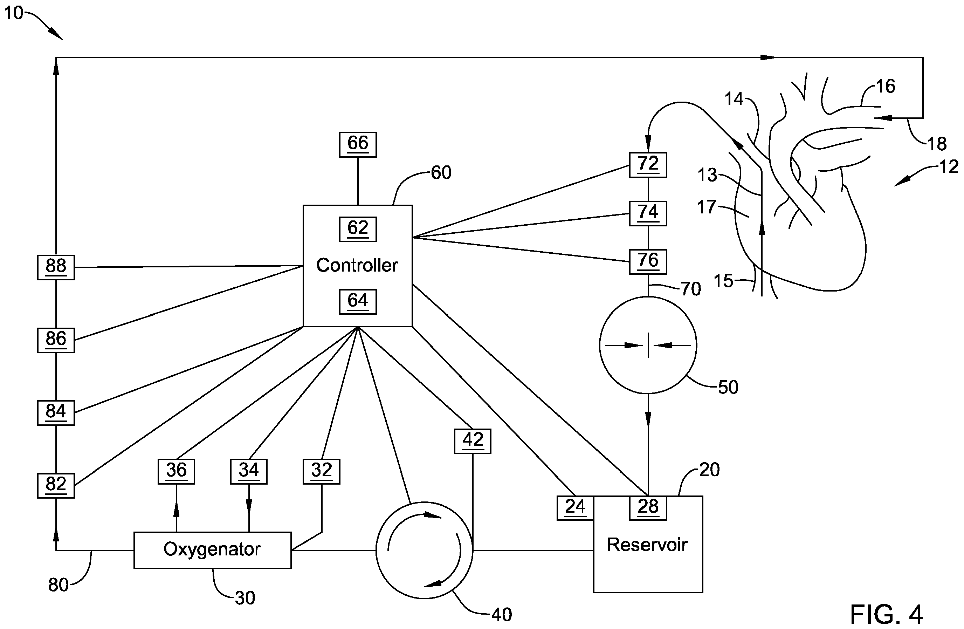

In some embodiments, the HLM system 10, the venous pathway 70, and/or the blood reservoir 20 may comprise a vacuum unit 28 disposed along, coupled to, and/or in communication with the venous pathway 70 and/or the blood reservoir 20, as seen in FIGS. 3-4. In some embodiments, the vacuum unit 28 may be in fluid communication with the venous pathway 70, the blood reservoir 20, and/or fluid (e.g., blood, priming fluid, etc.) disposed and/or flowing therein. In some embodiments, the vacuum unit 28 may be disposed upstream of the blood reservoir 20. In some embodiments, the vacuum unit 28 may be disposed adjacent to and/or in communication with the blood reservoir 20. In some embodiments, the vacuum unit 28 may be directly coupled to the blood reservoir 20. In some embodiments, the vacuum unit 28 may be disposed within the blood reservoir 20. In some embodiments, the vacuum unit 28 may be integrated with the blood reservoir 20. In some embodiments, the vacuum unit 28 may be spaced apart from the blood reservoir 20. Other configurations are also contemplated.

In some embodiments, the vacuum unit 28 may be in communication (e.g., electronic communication, wired communication, wireless communication, etc.) with the controller 60. In some embodiments, the vacuum unit 28 may be configured to generate negative pressure along and/or within the venous pathway 70 and/or the blood reservoir 20, thereby generating and/or enhancing the flow of fluid (e.g., blood, priming fluid, etc.) from the patient's vasculature and/or from the at least one venous portion of the patient's vasculature to the blood reservoir 20, and/or within the venous pathway 70.

In some embodiments, the vacuum unit 28 may be utilized in conjunction with a gravitational flow system, while in other embodiments, the vacuum unit 28 may be utilized without a gravitational flow system.

In some examples, the controller 60 may be configured to receive signals from the plurality of venous sensors and/or the blood level sensor 24 and adjust the vacuum unit 28 based on the signals received from the plurality of venous sensors and/or the blood level sensor 24. For example, a user may set the controller 60 to maintain a desired flow rate within the venous pathway 70. In response, the controller 60 may monitor signals received from the plurality of venous sensors and adjust the negative pressure generated by the vacuum unit 28 to maintain the flow rate with the venous pathway 70 at the desired level. Other configurations are also contemplated.

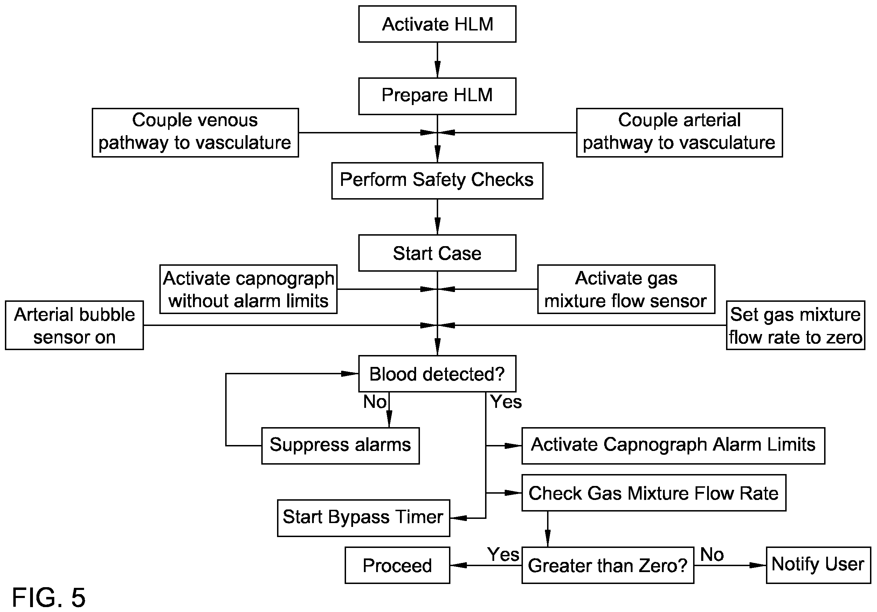

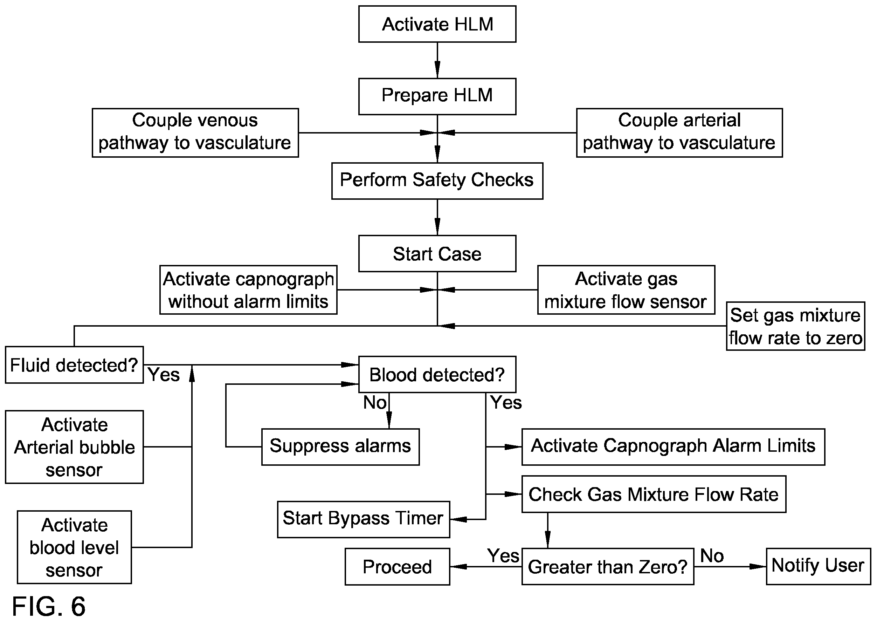

FIGS. 5-6 are flow charts illustrating selected aspects of a method of bypassing a patient's heart 12 with a heart-lung machine (HLM) system 10. In some embodiments, the method of bypassing the patient's heart 12 with the heart-lung machine (HLM) system 10 may comprise activating the HLM system 10. The HLM system 10 may comprise one or more components as described herein. In some at least some embodiments, the HLM system 10 may comprise the controller 60, and a plurality of venous sensors disposed along the venous pathway 70. In some embodiments, the plurality of venous sensors may comprise the venous blood gas sensor 72. Other sensors and/or components are also contemplated, as described herein. In at least some embodiments, when the HLM system 10 is activated, the controller 60 may be configured to display a home screen. For example, the controller 60 may be configured to display the home screen on the visual display 62 and/or the touch screen display.

When the HLM system 10 is activated, measured values associated with and/or received from the plurality of venous sensors, the plurality of arterial sensors, and/or other sensors described herein may be available immediately. In at least some embodiments, the measured values may be displayed by the controller 60 (on the visual display 62, for example). In some embodiments, when the HLM system 10 is activated, the venous pressure sensor 76, the arterial pressure sensor 86, the pre-oxygenator pressure sensor 32, the blood level sensor 24, the arterial bubble sensor 88, and/or the pump inlet pressure sensor 42 may be activated immediately. In some embodiments, when the HLM system 10 is activated, the arterial bubble sensor 88, the arterial blood gas sensor 82, the capnograph sensor 36, the gas mixture flow sensor 34, and/or the blood level sensor 24 may be left off and/or switched off (e.g., deactivated). In some embodiments, some, or in some embodiments all, of the plurality of venous sensors, the plurality of arterial sensors, and/or other sensors described herein may be activated when any fluid (e.g., blood, priming fluid, etc.) is detected within the venous pathway 70 by the venous blood gas sensor 72. In some embodiments, the blood level sensor 24 and/or the arterial bubble sensor 88 may be activated when any fluid (e.g., blood, priming fluid, etc.) is detected within the venous pathway 70 by the venous blood gas sensor 72. Other configurations and/or sensor activations and/or deactivations are also contemplated.

At least some, and in some embodiments all, of the various sensors associated with the HLM system 10 may have alarms (e.g., one alarm, one or more alarms, a plurality of alarms, etc.) associated therewith. The alarms may notify a user of an out-of-range condition such as the measured value exceeding a predetermined maximum value or the measured value falling below a predetermined minimum value. The alarms may be visual, audible, tactile, or other types of physical alarms, including combinations thereof. During set-up and preparation of the HLM system 10 (e.g., before connection to the patient's vasculature), the alarms may not be as relevant as during operation of the HLM system 10 and may sometimes be considered as false alarms. False alarms may sometimes be ignored or overlooked by the user. As such, reducing incidences of false alarms and/or suppressing alarms produced before using the HLM system 10 to actually treat a patient may be desirable.

In some embodiments, the method of bypassing the patient's heart 12 with the heart-lung machine (HLM) system 10 may comprise preparing the HLM system 10. After the HLM system 10 has been activated, the HLM system 10 may undergo a preparation phase. During the preparation phase and/or while preparing the HLM system 10, profile data and/or information specific to an upcoming procedure and/or patient may be entered and/or loaded into the controller 60. In some embodiments, the profile data and/or information specific to the upcoming procedure and/or patient may include patient height, patient weight, calculated blood flow rate, etc. In at least some embodiments, the preparation phase and/or preparing the HLM system 10 may be done away from the patient and/or operating room (e.g., "offline"). During the preparation phase and/or while preparing the HLM system 10, the various components and/or elements of the HLM system 10 may be primed with a biocompatible fluid. For example, the venous pathway 70, the arterial pathway 80, the blood reservoir 20, the pump 40, the oxygenator 30, etc. may be primed with the biocompatible fluid to remove air and/or to prevent air ingress. In some embodiments, the biocompatible fluid may include saline solution or ringer's solution. In some embodiments, the biocompatible fluid may include saline solution or ringer's solution combined with one or more additives (e.g., therapeutic agents) such as heparin, mannitol, bicarbonate, etc. Other configurations and/or additives are also contemplated.

In some embodiments, the method of bypassing a patient's heart 12 with a heart-lung machine (HLM) system 10 may comprise coupling a venous pathway 70 of the HLM system 10 to at least one venous portion of the patient's vasculature. In some embodiments, coupling the venous pathway 70 of the HLM system 10 to at least one venous portion of the patient's vasculature may comprise coupling the venous pathway 70 to the superior vena cava 14, the inferior vena cava 15, and/or the right atrium 17. In some embodiments, coupling the venous pathway 70 of the HLM system 10 to at least one venous portion of the patient's vasculature may comprise coupling the venous pathway 70 to a venous cannula or cannulas 13. In some embodiments, the venous cannula or cannulas 13 may be inserted into the superior vena cava 14, the inferior vena cava 15, and/or the right atrium 17 before coupling the venous cannula or cannulas 13 to the venous pathway 70. In some embodiments, the venous cannula or cannulas 13 may be inserted into the superior vena cava 14, the inferior vena cava 15, and/or the right atrium 17 after coupling the venous cannula or cannulas 13 to the venous pathway 70. Other configurations are also contemplated.

In some embodiments, the method of bypassing the patient's heart 12 with the heart-lung machine (HLM) system 10 may comprise coupling an arterial pathway 80 of the HLM system 10 to at least one arterial portion of the patient's vasculature. In some embodiments, coupling the arterial pathway 80 of the HLM system 10 to at least one arterial portion of the patient's vasculature may comprise coupling the arterial pathway 80 to the aorta 16. In some embodiments, coupling the arterial pathway 80 of the HLM system 10 to at least one arterial portion of the patient's vasculature may comprise coupling the arterial pathway 80 to an arterial cannula or cannulas 18. In some embodiments, the arterial cannula or cannulas 18 may be inserted into the aorta 16 before coupling the arterial cannula or cannulas 18 to the arterial pathway 80. In some embodiments, the arterial cannula or cannulas 18 may be inserted into the aorta 16 after coupling the arterial cannula or cannulas 18 to the arterial pathway 80. Other configurations are also contemplated.

In some embodiments, the method of bypassing the patient's heart 12 with the heart-lung machine (HLM) system 10 may comprise performing safety checks on the HLM system 10 and/or the various system components and/or sensors thereof. In some embodiments, the HLM system 10 and/or the controller 60 may be configured to guide and/or prompt the user to perform the safety checks. In some embodiments, the HLM system 10 and/or the controller 60 may be configured to guide and/or prompt the user through performing the safety checks. In some embodiments, the HLM system 10 and/or the controller 60 may perform the safety checks automatically. In some embodiments, the controller 60 may prompt the user to perform the safety checks and manually approve or input satisfactory checks. In some embodiments, the controller 60 may permit the user to manually bypass one or more safety checks and/or override an unsatisfactory safety check. In some embodiments, the controller 60 may perform the safety check in a semi-automatic manner incorporating a combination of automatic checks and manual user input. Other configurations are also contemplated.

In some embodiments, the method of bypassing the patient's heart 12 with the heart-lung machine (HLM) system 10 may comprise performing safety checks on the HLM system 10 and/or the various system components and/or sensors thereof before coupling the venous pathway 70 of the HLM system 10 to at least one venous portion of the patient's vasculature and/or before coupling the arterial pathway 80 of the HLM system 10 to at least one arterial portion of the patient's vasculature.

In some embodiments, the method of bypassing the patient's heart 12 with the heart-lung machine (HLM) system 10 may comprise starting a case on and/or within the controller 60. For the purpose of this disclosure, a "case" may be understood to be a procedure associated with a patient using the HLM system 10. In some embodiments, when the case is started within the controller 60, the arterial bubble sensor 88 may be automatically activated. In some embodiments, when the case is started within the controller 60, the venous blood gas sensor 72 may be automatically activated. In some embodiments, when the case is started within the controller 60, the arterial blood gas sensor 82 may be initially and/or automatically deactivated. In some embodiments, after the case is started within the controller 60 (but before blood is detected within the venous pathway 70 by the venous blood gas sensor 72), the arterial blood gas sensor 82 may be manually activated by the user. In some embodiments, when the case is started within the controller 60, the capnograph sensor 36 may be automatically activated without alarm limits (e.g., the capnograph sensor 36 provides measured values but the measured values are not compared to any alarm limits). In some embodiments, when the case is started within the controller 60, the gas mixture flow sensor 34 may be automatically activated. In some embodiments, when the case is started within the controller 60, the flow rate of the compressed air and oxygen gas mixture entering the oxygenator 30 may be set to zero liters per minute. In at least some embodiments, after the case is started within the controller 60, the HLM system 10 may be considered to be "online", even if it is not actively bypassing the patient's heart 12.

In some embodiments, the method of bypassing the patient's heart 12 with the heart-lung machine (HLM) system 10 may comprise suppressing alarms associated with the plurality of venous sensors until fluid (e.g., blood, priming fluid, etc.), or a particular type of fluid, such as blood, is detected within the venous pathway 70 by the venous blood gas sensor 72. In some embodiments, the method of bypassing the patient's heart 12 with the heart-lung machine (HLM) system 10 may comprise suppressing alarms associated with the plurality of arterial sensors until fluid (e.g., blood, priming fluid, etc.), or a particular type of fluid, such as blood, is detected within the venous pathway 70 by the venous blood gas sensor 72. For example, the alarms may be suppressed when priming fluid is detected within the venous pathway 70 and/or the arterial pathway 80 and blood is not detected within the venous pathway 70, but once blood is detected within the venous pathway 70, the alarms may no longer be suppressed.

In some embodiments, the method of bypassing the patient's heart 12 with the heart-lung machine (HLM) system 10 may comprise suppressing alarms associated with the gas mixture flow sensor 34 until fluid (e.g., blood, priming fluid, etc.), or a particular type of fluid, such as blood, is detected within the venous pathway 70 by the venous blood gas sensor 72. In some embodiments, the method of bypassing the patient's heart 12 with the heart-lung machine (HLM) system 10 may comprise, when the case is started within the controller prior to detection of blood within the venous pathway by the venous blood gas sensor, automatically activating the gas flow mixture sensor 34 configured to detect the flow rate of a compressed air and oxygen gas mixture flowing into the oxygenator 30 of the HLM system 10, and setting the flow rate of the compressed air and oxygen gas mixture entering the oxygenator 30 to zero liters per minute. In some embodiments, the method of bypassing the patient's heart 12 with the heart-lung machine (HLM) system 10 may further comprise, upon detection of fluid (e.g., blood, priming fluid, etc.), or a particular type of fluid, such as blood, within the venous pathway 70 by the venous blood gas sensor 72, automatically checking if the flow rate of the compressed air and oxygen gas mixture entering the oxygenator 30 is greater than zero liters per minute.

In some embodiments, the method of bypassing the patient's heart 12 with the heart-lung machine (HLM) system 10 may comprise activating a gas flow notification if the flow rate of the compressed air and oxygen gas mixture entering the oxygenator 30 is not greater than zero liters per minute. In some embodiments, the gas flow notification may be visual, audible, tactile, and/or other types of notifications. In some embodiments, the controller 60 may be configured to show, display, and/or communicate the gas flow notification to the user. In some embodiments, the gas flow notification may be shown, displayed, and/or communicated by the controller 60 and/or the visual display 62. Other configurations are also contemplated.

In some embodiments, the method of bypassing the patient's heart 12 with the heart-lung machine (HLM) system 10 may comprise suppressing alarms associated with the capnograph sensor 36 until fluid (e.g., blood, priming fluid, etc.), or a particular type of fluid, such as blood, is detected within the venous pathway 70 by the venous blood gas sensor 72. In some embodiments, the method of bypassing the patient's heart 12 with the heart-lung machine (HLM) system 10 may comprise automatically activating alarm limits associated with the capnograph sensor 36 configured to detect the exhaled carbon dioxide (ExCO2) content leaving the oxygenator 30 of the HLM system 10 upon detection of fluid (e.g., blood, priming fluid, etc.), or a particular type of fluid, such as blood, within the venous pathway 70 by the venous blood gas sensor 72.

In some embodiments, the method of bypassing the patient's heart 12 with the heart-lung machine (HLM) system 10 may comprise, after starting the case within the controller 60 and while suppressing alarms associated with the plurality of venous sensors, the plurality of arterial sensors, the capnograph sensor 36, the gas mixture flow sensor 34, , and/or other sensors, continuously checking and re-checking for the presence and/or detection of fluid (e.g., blood, priming fluid, etc.), or a particular type of fluid, such as blood, within the venous pathway 70 by the venous blood gas sensor 72.

In some embodiments, upon initial detection of fluid (e.g., blood, priming fluid, etc.), or a particular type of fluid, such as blood, is detected within the venous pathway 70 by the venous blood gas sensor 72, the controller 60 may activate a blood detection notification. In some embodiments, the method of bypassing the patient's heart 12 with the heart-lung machine (HLM) system 10 may comprise, upon initial detection of fluid (e.g., blood, priming fluid, etc.), or a particular type of fluid, such as blood, is detected within the venous pathway 70 by the venous blood gas sensor 72, activating the bypass timer 66 to document the amount of time that blood flow bypasses the patient's heart 12 while the HLM system 10 is active. In some embodiments, upon initial detection of fluid (e.g., blood, priming fluid, etc.), or a particular type of fluid, such as blood, is detected within the venous pathway 70 by the venous blood gas sensor 72, and/or after the controller 60 activates the blood detection notification, the bypass timer 66 may be activated manually by the user. Other configurations are also contemplated.

It should be understood that this disclosure is, in many respects, only illustrative. Changes may be made in details, particularly in matters of shape, size, and arrangement of steps without exceeding the scope of the disclosure. This may include, to the extent that it is appropriate, the use of any of the features of one example embodiment being used in other embodiments. The disclosure's scope is defined in the language in which the appended claims are expressed.

## Claims

1. A heart-lung machine (HLM) system, comprising:
a controller; and
a plurality of venous sensors disposed along a venous pathway, the plurality of venous sensors comprising a venous blood gas sensor;
wherein upon activation of the HLM system, the controller is configured to suppress alarms associated with the plurality of venous sensors until blood is detected within the venous pathway by the venous blood gas sensor.

2. The HLM system of claim 1, further comprising a plurality of arterial sensors disposed along an arterial pathway.

3. The HLM system of claim 2, wherein upon activation of the HLM system, the controller is configured to suppress alarms associated with the plurality of arterial sensors until blood is detected within the venous pathway by the venous blood gas sensor.

4. The HLM system of claim 1, further comprising an oxygenator, a capnograph sensor configured to detect an exhaled carbon dioxide content leaving the oxygenator, and a gas mixture flow sensor configured to detect a flow rate of a compressed air and oxygen gas mixture flowing into the oxygenator.

5. The HLM system of claim 4, wherein the controller is configured to automatically activate the capnograph sensor without alarm limits when a case is started within the controller.

6. The HLM system of claim 5, wherein the controller is configured to automatically activate alarm limits associated with the capnograph sensor upon detection of blood within the venous pathway by the venous blood gas sensor.

7. The HLM system of claim 4, wherein the controller is configured to automatically activate the gas mixture flow sensor and set the flow rate of the compressed air and oxygen gas mixture entering the oxygenator to zero liters per minute when a case is started within the controller.

8. The HLM system of claim 7, wherein upon detection of blood within the venous pathway by the venous blood gas sensor, the controller is configured to automatically check if the flow rate of the compressed air and oxygen gas mixture entering the oxygenator is greater than zero liters per minute and to activate a gas flow notification if the flow rate of the compressed air and oxygen gas mixture entering the oxygenator is not greater than zero liters per minute.

9. The HLM system of claim 7, wherein upon detection of blood within the venous pathway by the venous blood gas sensor, the controller is configured to automatically set the flow rate of the compressed air and oxygen gas mixture to a predetermined value.

10. A heart-lung machine (HLM) system, comprising:
a controller;
an oxygenator;
a venous pathway configured to transport deoxygenated blood from at least one venous portion of a patient's vasculature to the oxygenator;
an arterial pathway configured to transport oxygenated blood from the oxygenator to at least one arterial portion of the patient's vasculature; and
a plurality of venous sensors disposed along the venous pathway, the plurality of venous sensors comprising a venous blood gas sensor;
wherein the venous pathway and the arterial pathway cooperate to bypass blood flow through a patient's heart;
wherein the controller includes a bypass timer configured to document an amount of time that blood flow bypasses the patient's heart while the HLM system is active;
wherein the controller is configured to start the bypass timer upon initial detection of blood within the venous pathway by the venous blood gas sensor.

11. The HLM system of claim 10, wherein upon activation of the HLM system, the controller is configured to suppress alarms associated with the plurality of venous sensors until blood is detected within the venous pathway by the venous blood gas sensor.

12. The HLM system of claim 10, further comprising a capnograph sensor configured to detect an exhaled carbon dioxide content leaving the oxygenator, wherein the controller is configured to automatically activate alarm limits associated with the capnograph sensor upon detection of blood within the venous pathway by the venous blood gas sensor.

13. The HLM system of claim 10, wherein the controller is configured to start the bypass timer before the patient's heart is completely bypassed by the HLM system.

14. The HLM system of claim 10, wherein the controller is configured to automatically stop the bypass timer when the HLM system is deactivated.
